Europäisches Patentamt

(19) European Patent Office

Office européen des brevets

(11) Publication number: 0 281 270 B1

## (12) EUROPEAN PATENT SPECIFICATION

(45) Date of publication of patent specification: **06.05.92**    (51) Int. Cl.⁵: **A61M 5/162**

(21) Application number: **88301256.9**

(22) Date of filing: **16.02.88**

(54) **Vented spike connection component.**

(30) Priority: **03.03.87 US 21181**

(43) Date of publication of application:
**07.09.88 Bulletin 88/36**

(45) Publication of the grant of the patent:
**06.05.92 Bulletin 92/19**

(84) Designated Contracting States:
**AT BE CH DE ES FR GB GR IT LI LU NL SE**

(56) References cited:
**FR-A- 245 223**
**GB-A- 1 256 419**
**GB-A- 2 171 678**
**US-A- 2 409 343**
**US-A- 2 817 372**

(73) Proprietor: **SHERWOOD MEDICAL COMPANY**
**1915 Olive Street**
**St. Louis, MO 63103(US)**

(72) Inventor: **d'Alo, Herbert Fred**
**37 Forest Hill Drive**
**Madison Connecticut 06443(US)**
Inventor: **Enger, Christine Dale**
**27 Demott Street**
**Tenafly New Jersey 07670(US)**

(74) Representative: **Porter, Graham Ronald et al**
**C/O John Wyeth & Brother Limited Huntercombe Lane South Taplow**
**Maidenhead Berkshire, SL6 0PH(GB)**

## Description

The present invention relates to a fluid container and connection component arranged for mounting on a tubing assembly, the container being a prefilled, foil-sealed container, the connection component including a spike for penetrating a foil seal and an air vent.

In an enteral fluid delivery system for a patient, there is a need to provide a component that will effect a quick connection of a fluid delivery set to a prefilled, foil-sealed container containing enteral nutritional fluid. In such systems the connecting component is preferably in the form of a cap, which replaces the shipping cap on the prefilled container, and includes means for perforating the foil seal on the container during attachment. Such automatic perforation is desirable to simplify the set-up procedure. It is further desirable that the connecting component provide a means to allow air to vent into the container as fluid flows from the container. Such venting should be achieved without permitting venting air bubbles from entering the outlet flow channel.

GB-A-1256419 describes a parenteral solution container and delivery cap therfor, the delivery cap comprising a top portion and a skirt portion, the top portion having a passage for parenteral solution, the end of the passage outside the skirt being adapted for connection to a delivery tube, the end inside the skirt leading to a hollow needle for puncturing a sealing diaphragm when the latter is positioned in the neck of the parenteral solution container, the skirt portion being adapted for engagement with the neck of the container and carrying a flexible sleeve, one end of the sleeve being attached rigidly to the skirt, the other end having an inwardly directed flange, the neck of the container being adapted for co-operating engagement with the skirt and the container having an outwardly directed flange for snap engagement with the flange of the flexible sleeve.

Accordingly, the present invention seeks to provide a fluid container and connection component for effecting a quick coupling between a fluid delivery set and the prefilled, sealed container that minimizes the time of exposure of the container contents between seal puncture and attachment to the fluid delivery set.

This invention also seeks to provide such an connection component and prefilled, foil-sealed container that inhibits entry of air into the fluid flow path.

Further this invention seeks to provide such an arrangement in which the connection component can puncture and deform the seal of the prefilled bottle in a manner that permits the venting of air into the container and the pumping of fluid therefrom.

The invention provides a fluid container and connection component wherein the fluid container has a pierceable protective seal covering the opening and a threaded connection for receiving a closure cap comprising a cap shaped component body including a circular wall portion and a cylindrical threaded portion extending in one axial direction therefrom, said threaded portion being adapted to threadedly receive the threaded connection on the container, a pointed projecting member extending from the wall portion, the projecting member having a fluid receiving passage passing through the wall portion on the same side as the threaded portion and having a fluid receiving opening on the side of said projecting member facing away from the centre and an air vent situated on the wall portion, characterised in that said member has a centreline laterally offset from the centreline of said wall portion on the same side as the threaded portion, and that the air vent is spaced on a side laterally opposite to said pointed projecting member, the end of said member being pointed such that in use the pointed end pierces the seal of the container to form an aperture in the seal larger than the diameter of the projecting member upon threaded connection of said connection component to the container.

Preferably the air vent is arranged on the wall spaced from the projecting member on the opposite side of the centre.

For a better understanding of the invention, as well as other objects and further features thereof, reference is had to the following detailed description of the preferred embodiments which makes reference to the following set of drawings.

## BRIEF DESCRIPTION OF THE DRAWINGS

Fig. 1 is a perspective assembly view, partly in section along the line 1 - 1 of Fig. 2, of a connection component according to the present invention

Fig. 2 is a top view of the connection component shown In Fig. 1;

Fig. 3 is a top view of a foil seal of a prefilled container after the seal has been deformed by the attachment of the Fig. 1 connection component to said container

Fig. 4 is a cut-away view after the connection component has been attached to the container as in Fig. 5;

Fig. 5 is a perspective assembly view, partly in section, of the Fig. 1 connection component in operative engagement with a prefilled container.

## DETAILED DESCRIPTION OF THE PREFERRED EMBODIMENTS

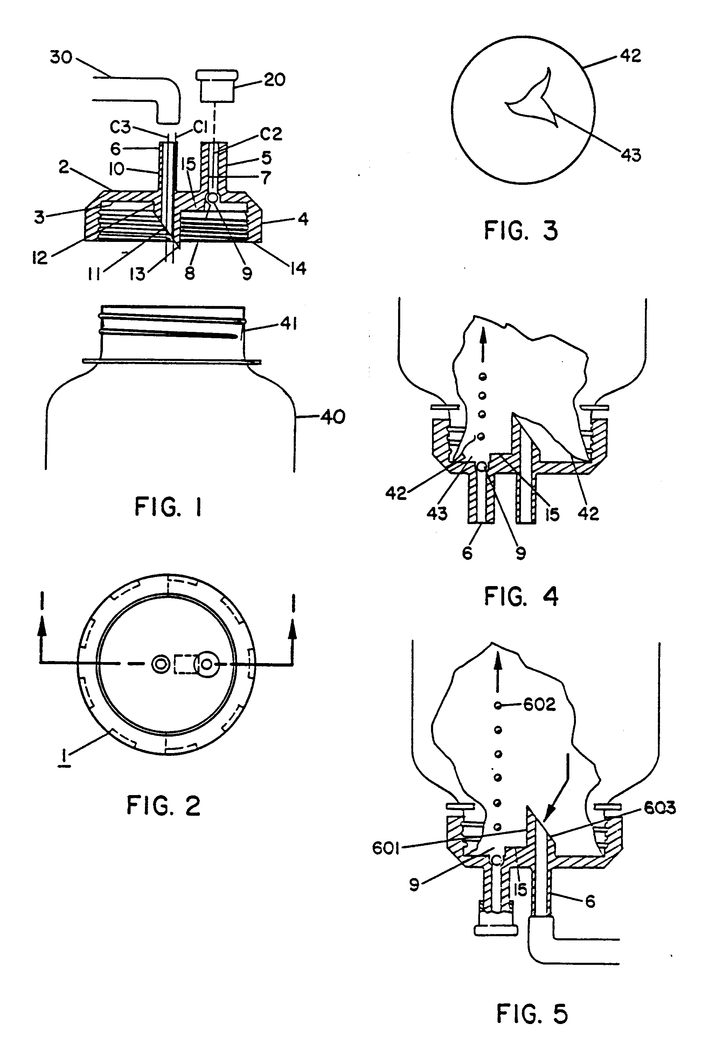

Referring to Figs. 1 and 2 the vented spike connection component of the present invention is generally comprised of a housing body 1 including a circular wall having a top surface 2, a bottom innerside surface 3, and a rim 4 adapted to be detachably mounted to the neck 41 of a prefilled container 40. The connection component is preferably formed from moulded plastic and has a one piece construction.

The housing body 1 is provided with two cylindrically shaped members 5 and 6 extending outward from surface 2. Member 5 has an internal passage of which forms an air vent and includes a one way valve 9 arranged within recess 8 formed as an enlargement of the inner end of passage 7. A cover 20 may be provided to close passage 7 prior to use.

Cylindrical member 6 includes a top portion 10 extending outward from surface 2 and is adapted to be attached to a plastic tubing 30 which along with the connection component forms an enteral fluid delivery set. Cylindrical member 6 also includes a bottom portion 11 having a spike formed by truncating the cylindrical member portion 11 with an intersecting angled plane starting at a location 12 just below the bottom surface 3 of the housing body 1 to a point 13 extending below the edge 14 of the rim 4. Member 6 includes a central passage through which enteral fluid can pass from container 40 into tubing 30.

In the Fig. 1 embodiment, the circular wall formed between surfaces 2 and 3 has a central axis designated C1 about which the connection component is rotated for attachment to fluid container bottle 40. In the illustrated embodiment cylindrical member 5 and 6 are each offset from axis C1 on opposite sides of C1. In addition the shape of the spike 13 formed on portion 11 of cylindrical member 6 is oriented so that the opening of the central passage is facing away from the vent passage 7 formed through member 5. the centre C3 of member 6 is offset by approximately 0.1 inch (2.5 mm) from the centre C1 of the connection component. This offset provides for effective action of the spike end 13 in opening a passage through a foil seal on a fluid container 40.

The centre C3 of member 6 is offset by approximately 0.5 inch (12.7 mm) from the centre C2 of member 5. This separation, along with the orientation of the opening of the passage in member 6 prevents air bubbles which enter fluid via vent passage 7 from being drawn into the outlet passage in member 6. Additionally, a rib 15 is provided on the bottom innerside surface 3 of the connection component. The rib 15 acts to deflect the seal after it has been punctured, thereby prohibiting the seal from obstructing the air passage in member 5.

With the aforedescribed vented spike connection component of the present invention, possible interference caused by collection of the seal of the container around the base of the projecting member, after it has pierced the seal of the container, is eliminated by the deformation of the seal in a manner as shown in Fig. 3. This irregularly shaped aperture 43 in the seal 42 is caused by the offset of its centreline C3 of cylindrical member 6 from the axis C1 of the housing. With reference to Fig. 4, the aperture 43 is sufficiently extended to permit air to be vented into the container without being obstructed by the foil seal 42. Also the aperture is sufficiently deformed at the centre as to allow clear passage of the fluid to the fluid carrying projecting member 6.

When in use the connection component according to the present invention disposes the long side 601 of the fluid carrying projecting member 6 adjacent the vented air stream 602 as illustrated in Fig. 5. This placement provides a physical barrier between the vented air stream 602 and the area adjacent the point of fluid exit 603. Coupled with a sufficient distance displacement between the air stream 602 and the fluid exit point 603 they form a system that provides reliable delivery of the fluid from the container to the fluid flow circuit without inducement of air into the circuit in the form of a bubble. Further, the flow of fluid or vent air is not obstructed by the foil seal.

The detailed description of the preferred embodiment of the invention having been set forth herein for the purpose of explaining the principles thereof, it is known that there may be modification, variation or change in the invention without departing from the proper scope of the invention and the claims thereto.

## Claims

1. A fluid container and connection component wherein the fluid container has a pierceable protective seal (42) covering the opening and a threaded connection for receiving a closure cap comprising a cap shaped component body (1) including a circular wall portion (2,3) and a cylindrical threaded portion extending in one axial direction therefrom, said threaded portion being adapted to threadedly receive the threaded connection on the container, a pointed projecting member (6) extending from the wall portion (2,3), the projecting member (6) having a fluid receiving passage passing through the wall portion (2,3) on the same side as the threaded portion and having a fluid receiving opening on the side of said projecting member (6) facing away from the centre and an air vent (5) situated on the wall portion, characterised in that said member (6) has a centreline (C3) laterally offset from the centreline (C1) of said wall portion (2,3) on the same side as the threaded portion, and that the air vent (5) is spaced on a side laterally opposite to said pointed projecting member (6), so that in use the pointed end (13) pierces the seal (42) of the container to form an aperture (43) in the seal larger than the diameter of the projecting member (6) upon threaded connection of said connection component to the container.

2. A fluid container and connection component as claimed in Claim 1 wherein the projecting member (6) is cylindrical and has a pointed end formed by an intersecting angled plane.

3. A fluid container and connection component as claimed in Claim 1 or Claim 2, wherein the projecting member (6) is spaced from the centre by at least approximately one tenth of an inch (2.5mm).

4. A fluid container and connection component as claimed in any one of the preceding Claims wherein the projecting member (6) is spaced from the air vent (5) by approximately one half of an inch (12.7mm).

5. A fluid container and connection component as claimed in any one of the preceding Claims wherein said air vent includes a one-way valve.

6. A fluid container and connection component as claimed in any one of the preceding Claims further comprising a rib member displaced on the wall portion (3) between the projecting member (6) and the air vent (5).

7. A fluid container and a connection component as claimed in any one of the preceding Claims wherein the pointed end (13) extends further toward the container than any portion of the fluid receiving opening and the pointed end (13) is disposed between the fluid receiving opening and the vent opening.

8. A fluid container and a connection component as claimed in Claim 7, wherein all the fluid receiving opening extends substantially further toward the container than any portion of the vent opening.

**Revendications**

1. Récipient de liquide à élément de raccordement dans lequel le récipient de liquide comporte un opercule de protection perçable (42) qui couvre son orifice et un goulot fileté destiné à recevoir un couvercle de bouchage comprenant un corps formant couvercle (1) qui comporte une paroi circulaire (2, 3) et une partie cylindrique taraudée s'étendant dans un sens axial, la partie taraudée étant à même de se visser sur le goulot fileté du récipient, un élément saillant pointu (6) partant de la paroi (2, 3), cet élément saillant (6) comportant un passage pour le liquide qui traverse la paroi (2, 3) du même côté que la partie taraudée et présente un orifice à liquide du côté de l'élément saillant (6) opposé au centre et un évent de prise d'air (5) situé sur la paroi, caractérisé en ce que l'élément (6) présente un axe central (C3) décalé latéralement de l'axe central (C1) de la paroi (2, 3) du même côté que la partie taraudée, et que l'évent de prise d'air (5) est espacé d'un côté latéralement opposé à l'élément saillant pointu (6), de sorte qu'en service l'extrémité pointue (13) perce l'opercule (42) du récipient pour former, dans cet opercule, une ouverture (43) plus grande que le diamètre de l'élément saillant (6), lors du vissage de l'élément de raccordement sur le récipient.

2. Récipient de liquide à élément de raccordement suivant la revendication 1, dans lequel l'élément saillant (6) est cylindrique et comporte une extrémité pointue formée par un plan oblique de coupe.

3. Récipient de liquide à élément de raccordement suivant la revendication 1 ou 2, dans lequel l'élément saillant (6) est espacé du centre d'au moins environ 2,5 mm (0,1 pouce).

4. Récipient de liquide à élément de raccordement suivant l'une quelconque des revendications précédentes, dans lequel l'élément saillant (6) est espacé de l'évent de prise d'air (5) d'environ 12,7 mm (0,5 pouce).

5. Récipient de liquide à élément de raccordement suivant l'une quelconque des revendications précédentes, dans lequel l'évent de prise d'air comprend un clapet de retenue.

6. Récipient de liquide à élément de raccordement suivant l'une quelconque des revendications précédentes, comprenant, en outre, une nervure décalée sur la partie de paroi (3) entre l'élément saillant (6) et l'évent de prise d'air (5).

7. Récipient de liquide à élément de raccordement suivant l'une quelconque des revendications précédentes, dans lequel l'extrémité pointue (13) s'étend davantage vers le récipient que n'importe quelle partie de l'orifice à liquide et l'extrémité pointue (13) est disposée entre l'orifice à liquide et l'orifice de l'évent.

8. Récipient de liquide à élément de raccordement suivant la revendication 7, dans lequel l'entièreté de l'orifice à liquide s'étend en substance davantage vers le récipient que n'importe quelle partie de l'orifice de l'évent.

**Patentansprüche**

1. Flüssigkeitsbehälter und Verbindungselement, wobei der Flüssigkeitsbehälter einen seine Öffnung verschließenden, durchstoßbaren Schutzverschluß (42) und ein Gewinde zur Aufnahme einer Verschlußkappe aufweist, die einen kappenförmigen Verbindungselement-Körper (1) mit einem kreisförmigen Wandabschnitt (2,3) und einem zylindrischen, axial gerichteten Gewindeabschnitt umfaßt, wobei der Gewindeabschnitt zum Eingriff in das Gewinde am Behälter eingerichtet ist und aus dem Wandabschnitt (2,3) ein spitzer Vorsprung (6) ragt, wobei der Vorsprung (6) einen auf der Seite des Gewindeabschnitts durch den Wandabschnitt (2,3) führenden Durchflußweg mit einer Einlaßöffnung aufweist, die an der Seite des Vorsprungs (6) angeordnet ist und vom Mittelpunkt und einer am Wandabschnitt befindlichen Entlüftungsöffnung (5) abgewandt ist,
**dadurch gekennzeichnet,** daß
die Mittellinie (C3) des Vorsprungs (6) auf der Seite des Gewindeabschnitts gegenüber der Mittellinie (C1) des Wandabschnitts (2,3) seitlich versetzt ist, und daß die Entlüftungsöffnung (5) im Abstand von dem spitzen Vorsprung (6) und seitlich entgegengesetzt zu ihm angeordnet ist, so daß im Gebrauch das spitze Ende (13) den Verschluß (42) des Behälters durchstößt, um beim Aufschrauben des Verbindungselements auf den Behälter eine Öffnung (43) in dem Verschluß auszubilden, die größer als der Durchmesser des Vorsprungs (6) ist.

2. Flüssigkeitsbehälter und Verbindungselement nach Anspruch 1, wobei der Vorsprung (6) zylindrisch ausgebildet ist und ein durch eine abgewinkelte Schnittebene gebildetes spitzes Ende aufweist.

3. Flüssigkeitsbehälter und Verbindungselement nach Anspruch 1 oder 2, wobei der Vorsprung (6) vom Mittelpunkt mindestens ungefähr einen Zehntel Inch (2,5 mm) beabstandet ist.

4. Flüssigkeitsbehälter und Verbindungselement nach einem der vorhergehenden Ansprüche, wobei der Vorsprung (6) von der Entlüftungsöffnung (5) ungefähr einen halben Inch (12,7 mm) beabstandet ist.

5. Flüssigkeitsbehälter und Verbindungselement nach einem der vorhergehenden Ansprüche, wobei die Entlüftungsöffnung ein Rückschlagventil umfaßt.

6. Flüssigkeitsbehälter und Verbindungselement nach einem der vorhergehenden Ansprüche, weiter umfassend eine auf dem Wandabschnitt (3) zwischen dem Vorsprung (6) und der Entlüftungsöffnung (5) angeordnete Rippe.

7. Flüssigkeitsbehälter und Verbindungselement nach einem der vorhergehenden Ansprüche, wobei das spitze Ende (13) weiter in Richtung Behälter ragt als irgend ein anderes Teil der Einlaßöffnung, und das spitze Ende (13) zwischen der Einlaßöffnung und der Entlüftungsöffnung angeordnet ist.

8. Flüssigkeitsbehälter und Verbindungselement nach Anspruch 7, wobei die ganze Einlaßöffnung wesentlich weiter in Richtung Behälter ragt als irgend ein Teil der Entlüftungsöffnung.

FIG. 3

FIG. 1

FIG. 4

FIG. 2

FIG. 5